# EUROPEAN PATENT SPECIFICATION

(11) **EP 0 389 286 B1**
(45) Date of publication and mention of the grant of the patent: **10.07.1996**
(21) Application number: 90303108.6
(22) Date of filing: 22.03.1990
(51) Int. Cl.: A61K 39/245, C07K 14/045, C12N 15/38

(54) **Recombinant cytomegalovirus vaccine**
Rekombinanter Cytomegalovirus-Impfstoff
Vaccin recombinant contre le virus cytomégalo

(30) Priority: 24.03.1989 US 328406
(43) Date of publication of application: 26.09.1990
(73) Proprietor: THE WISTAR INSTITUTE OF ANATOMY AND BIOLOGY, Philadelphia, PA 19104 (US)
(72) Inventor: Plotkin, Stanley A., Pennwynne, PA 19151 (US); Ricciardi, Robert P., Drexell Hill, PA 19026 (US); Gonczol, Eva, Rosemont, PA 19010 (US)
(74) Representative: Hale, Stephen Geoffrey

(56) References cited:
- EP-A- 0 180 288
- EP-A- 0 236 145
- EP-A- 0 252 302
- EP-A- 0 252 531
- EP-A- 0 268 014
- EP-A- 0 271 201
- EP-A- 0 277 773

## Description

The present invention relates generally to a recombinant human cytomegalovirus vaccine, and more specifically to a subunit vaccine containing the HCMV major glycoprotein complex subunit gene, gB.

Cytomegalovirus (CMV) is one of a group of highly host-specific herpes viruses that produce unique large cells bearing intranuclear inclusions. Human cytomegalovirus (HCMV) is characterized by a major glycoprotein complex variously termed gB, gA or gCI. HCMV causes cytomegalic inclusion disease and has been associated with a syndrome resembling infectious mononucleosis.

CMV infection in utero is an important cause of central nervous system damage in newborns. Although the virus is widely distributed in the population, about 40% of women enter pregnancy without antibodies and thus are susceptible to infection. About 1% of these women undergo primary infection in utero. Classical cytomegalic inclusion disease is rare; however, a proportion of the infected infants, including those who were symptom-free, are subsequently found to be mentally retarded.

Preliminary estimates based on surveys of approximately 4,000 newborns from several geographical areas indicate that the virus causes significant damage of the central nervous system leading to mental deficiency in at least 10%, and perhaps as high as 25%, of infected infants. Assuming that about 1% of newborn infants per year excrete CMV and that about one fourth of those develop mental deficiency, in the United States this means approximately 10,000 brain-damaged children born per year. This is a formidable number, particularly in view of the ability of these children to survive [J. Infect. Dis. 123 (5):555 (1971)].

HCMV in humans has also been observed to cause serious complications and infections in the course of organ transplants, especially with kidney and liver transplants.

Several HCMV vaccines have been developed or are in the process of development. Vaccines based on live attenuated strains of HCMV have been described. [See, e.g., S.A. Plotkin et al, Lancet, 1:528-30 (1984); S.A. Plotkin et al, J. Infect. Dis., 134:470-75 (1976); S.A. Plotkin et al, "Prevention of Cytomegalovirus Disease by Towne Strain Live Attenuated Vaccine", in Birth Defects, Original Article Series, 20(1):271-87 (1984); J.P. Glazer et al, Ann.Intern. Med., 91:676-83 (1979); and U.S. Patent 3,959,466.] A proposed HCMV vaccine using a recombinant vaccinia virus expressing HCMV glycoprotein B has also been described. [See, e.g., M.P. Cranage et al, EMBO J., 5:3057-63 (1986).] However, vaccinia models for vaccine delivery are believed to cause local reactions. Additionally, vaccinia vaccines are considered possible causes of encephalitis.

Adenoviruses have been developed previously as efficient heterologous gene expression vectors. For example, an adenovirus vector has been employed to express herpes simplex virus glycoprotein gB [D.C. Johnson et al, Virol., 164:1-14 (1988)]; human immunodeficiency virus type 1 envelope protein [R.L. Dewar et al, J. Virol., 63:129-36 (1988)]; and hepatitis B surface antigen [A.R. Davis et al, Proc. Natl. Acad. Sci., U.S.A., 82:7560-4 (1985); J.E. Morin et al, Proc. Natl. Acad. Sci., U.S.A., 84:4626-30 (1987)]. Adenoviruses have also been found to be non-toxic as vaccine components in humans. [See, e.g., E.T. Takajuji et al, J. Infect. Dis., 140:48-53 (1970); P.B. Collis et al, J. Infect. Dis., 128:74-750 (1973); and R.B. Couch et al, Am. Rev. Respir. Dis., 88:394-403 (1963).]

EP-A-0,236,145 refers to glycoproteins B and H of the human cytomegalovirus (HCMV) and the use of these glycoproteins in vaccines or in the purification and detection of HCMV antibody.

EP-A-0,180,288 refers to a subunit vaccine using glycoprotein A of HCMV, which is particularly useful in immunosuppressed individuals and women of child-bearing age.

EP-A-0,252,302 refers to the production of a 58 kD glycoprotein of HCMV by expression of a gene located in an open reading frame in the Hind IIIF fragment of the HCMV strain Ad169.

There remains a need in the art for additional vaccines capable of preventing CMV infection by generating neutralizing antibody and cellular responses to CMV in the human immune system.

In one aspect, the present invention provides a non-defective recombinant adenovirus genetically engineered to contain a DNA sequence encoding a human cytomegalovirus (CMV) subunit protein under the control of an expression control sequence, said adenovirus being capable of expressing said subunit in a host cell or a human.

Another aspect of the invention is a method for preparing a non-defective recombinant adenovirus for use in a subunit vaccine capable of eliciting an immune response to CMV upon administration to a human,
said method for preparing comprising the steps of :-
(a) inserting by genetic engineering techniques a DNA sequence encoding a human cytomegalovirus (CMV) subunit protein into an adenovirus strain under the control of an expression control sequence, said adenovirus being capable of expressing said subunit in a host cell;
(b) transfecting said recombinant adenovirus into a host cell;
(c) culturing said host cell in which the recombinant adenovirus expresses the CMV subunit in vitro;
(d) isolating therefrom a recombinant CMV subunit protein, and
(e) admixing said subunit protein with a suitable pharmaceutical carrier.

Yet another aspect of the invention is a method for preparing a non-defective recombinant adenovirus for use in a vaccine capable of eliciting an immune response to CMV upon administration to a human,
said method comprising the steps of:
(a) inserting by genetic engineering techniques a DNA sequence encoding a human cytomegalovirus (CMV) subunit protein into an adenovirus strain under the control of an expression control sequence, said adenovirus being capable of expressing said subunit in a host cell or human; and
(b) admixing said recombinant adenovirus with a pharmaceutically acceptable carrier.

A further aspect of the present invention is a vaccine comprising a genetically engineered non-defective recombinant adenovirus according to the invention in a pharmaceutical carrier, said vaccine being capable of eliciting an immune response to CMV upon administration to a human. Such a vaccine is capable of replicating in vivo, producing the CMV subunit protein in the vaccinated patient.

Thus, as still a further aspect, the invention provides for the use of the adenovirus to prepare a pharmaceutical composition which generates an immune response to CMV in vivo in the patient. Such immune response is capable of providing protection against exposure to the whole human CMV microorganism.

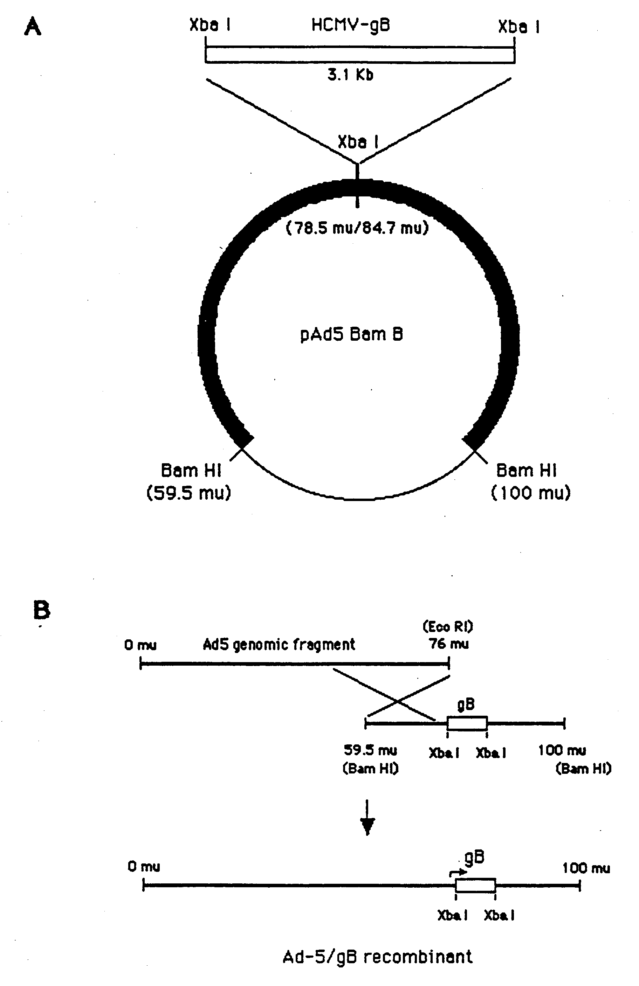

In the accompanying drawings Fig. 1 illustrates diagrammatically the construction of the recombinant adenovirus virus Ad5/gB, containing the gB gene of the Towne strain of HCMV described in Example 1 below. In the Figure, the restriction enzymes are identified as follows: X is XbaI, B is BamHI.

Fig. 1A illustrates cloning of the gB gene into the early region 3 (E3) transcription unit of Ad5. Represented are the 3.1kb fragment containing the gB gene by the open box; the adenovirus sequences extending from 59.5 to 100 mu (except for the deletion of the 78.5 to 84.7 mu length) by the filled portion of the circle; and the large BamHI fragment of the pBR322 by the thin line of the circle.

Fig. 1B illustrates the construction of the Ad5/gB recombinant virus. This figure shows the 59.5 mu to 76 mu region where homologous recombination occurs (as indicated by the crossed lines) between wild-type Ad5 viral sequence and the adenovirus sequences present on the pAd5 plasmid containing the gB gene. The plaque-purified recombinant virus retains the cloning XbaI sites and the direction of transcription of the gB gene from the E3 promoter is indicated by the bent arrow.

The present invention provides an adenovirus expression system capable of expressing an HCMV subunit gene for use as a vaccine against HCMV. Although any adenovirus strain capable of replicating in mammalian cells in vitro may be used to construct the expression vector for the subunit, a preferred expression system involves a non-defective adenovirus. Amongst such useful strains is adenovirus type 5. Alternatively, other desirable adenovirus strains may be employed which are capable of being orally administered, for use in expressing the CMV subunit in vivo. Such strains useful for in vivo production of the subunit include adenovirus 5 strains, and adenovirus type 4, 7 and 21 strains. [See, e.g., Takajuju et al, cited above). Appropriate strains of adenovirus, including those identified above and those employed in the Examples below are publicly available from sources such as the American Type Culture Collection (ATCC), Rockville, Maryland, USA.

Similarly, in the construction of the adenovirus vector, any of the subunits of the HCMV envelope protein may be employed. The presently preferred subunit protein is the HCMV gB subunit. For example, a replication-competent adenovirus-5 vector may be provided carrying the HCMV gB for expression in viral cultures. In the practice of this invention the HCMV subunit may be produced by recombinant techniques in large quantities sufficient for use in a subunit vaccine.

In a manner similar to the use of the gB subunit in this vaccine, other subunits of CMV which may be employed in the production of a vaccine according to the invention may be selected from the gA/gB, or gcII or gcIII or immediate early subunits of the human virus. Alternatively, more than one of these subunits may be employed in a vaccine according to the teachings of the present invention.

A number of strains of human CMV have been isolated. For example, the Towne strain of CMV, a preferred strain for use in preparation of the vaccine because of its broad antigenic spectrum and its attenuation, was isolated from the urine of a two-month-old male infant with cytomegalic inclusion disease (symptoms - central nervous system damage and hepatosplenomegaly). This strain of CMV was isolated by Stanley A. Plotkin, M.D., of The Wistar Institute of Anatomy and Biology, Philadelphia, Pennsylvania, USA, and is described in J. Virol., 11 (6): 991 (1973). This strain is freely available from The Wistar Institute or from the ATCC. However, other strains of CMV useful in the practice of this invention may be obtained from depositories like the ATCC or from other Institutes or Universities.

In addition to isolating the desired subunit from an available strain of HCMV, the sequences of the subunits of two strains have been published [See, e.g., Mach et al, J. Gen. Virol., 67:1461-67 (1986); Cranage et al, (1986) cited above; and Spaete et al, Virol., 167:207-25 (1987).] These subunit sequences can therefore be chemically synthesized by conventional methods known to ones skilled in the art, or the sequences purchased from commercial sources.

The recombinant virus of the present invention may also contain multiple copies of the HCMV subunit. Alternatively, the recombinant virus may contain more than one HCMV subunit type, so that the virus may express two or more HCMV subunits or immediate-early antigens and subunits together.

In the construction of the adenovirus vector the CMV subunit sequence is preferably inserted in an adenovirus strain under the control of an expression control sequence in the virus itself. The adenovirus vector preferably contains other sequences of interest in addition to the HCMV subunit. Such sequences may include regulatory sequences, enhancers, suitable promoters, secretory signal sequences and the like. The techniques employed to insert the subunit sequence into the adenovirus vector and make other alterations in the viral DNA, e.g. to insert linker sequences and the like, are known to ones skilled in the art. See, e.g., T. Maniatis et al, "Molecular Cloning, A Laboratory Manual", Cold Spring Harbor Laboratory, Cold Spring Harbor, NY (1982). Thus, the construction of suitable adenovirus expression vectors for in vitro expression of an HCMV subunit protein is within the skill of the art.

Once the recombinant viral vector containing the CMV subunit protein is constructed, it may be infected into a suitable host cell for in vitro expression. The infection of the recombinant viral vector is performed in a conventional manner. [See, Maniatis et al, cited above.] Suitable host cells include mammalian cells or cell lines, e.g. VERO or 293 (human embryonic kidney) cells.

The host cell, once infected with the recombinant virus of the present invention, is then cultured in a suitable medium, such as Minimal Essential Medium (MEM) for mammalian cells. The culture conditions are conventional for the host cell and allow the subunit to be produced either intracellularly or secreted extracellularly into the medium. Conventional protein isolation techniques are employed to isolate the expressed subunit from the selected host cell or medium.

When expressed in vitro and isolated from culture, the subunit may then be formulated into an appropriate vaccine composition. Such compositions will generally contain one or more of the recombinant CMV subunits. The preparation of a pharmaceutically acceptable vaccine composition, having appropriate pH, isotonicity, stability and other conventional characteristics is within the skill of the art. Thus such vaccines may optionally contain other components, such as adjuvants and/or carriers, e.g. aqueous suspensions of aluminum and magnesium hydroxides.

Alternatively, the vaccine composition of the present invention consists of the recombinant adenovirus itself, constructed as described above. Rather than infecting a host cell with the recombinant virus, the recombinant virus is introduced directly into the patient by vaccination. The recombinant virus, when introduced into a patient directly, infects the patient's cells and produces the CMV subunit in the patient's cells.

Thus, humans may be vaccinated against human CMV infection with one or more recombinantly-produced human CMV subunit proteins. The in vitro produced subunit proteins may be introduced into the patient in a vaccine composition as described above, preferably employing the oral, nasal or subcutaneous routes of administration. Where the recombinant virus is employed as the vaccine, it is preferably orally administered, because the adenoviruses are known to replicate in cells of the stomach. Previous studies with adenoviruses have shown them to be safe when administered orally. [See, e.g., Collis et al, cited above.] However, the present invention is not limited by the route of administration selected for the vaccine.

The dosage for all routes of administration of the in vitro vaccine containing one or more of the CMV subunit proteins is generally greater than 20 micrograms of protein per person, and preferably between 40 and 80 micrograms of protein per person. When the recombinant virus is itself administered as the vaccine, a dosage of between 10⁵ and 10⁸ plaque-forming units may be used. Additional doses of the vaccines of this invention may also be administered where considered desirable by the physician. The dosage regimen involved in the method for vaccination against CMV infection with the vaccine compositions and recombinant virus of the present invention will be determined considering various clinical and environmental factors known to affect vaccine administration.

The following Examples illustrate the construction of a non-defective adenovirus strain capable of expressing the HCMV major envelope glycoprotein subunit gB, and the efficacy of this composition as an exemplary HCMV vaccine. These Examples are illustrative only and do not limit the scope of the present invention.

### Example 1 - Construction of a Non-defective Adenovirus - gB Recombinant

The gB gene was cloned from the Towne strain of HCMV [Wistar Institute] as follows. The gB gene was first mapped to the 20.5 kb Hind III D fragment of HCMV using oligonucleotides that corresponded to the 5' and 3' termini of the published AD-169 gB sequence [See Cranage et al (1986), cited above]. The Hind III fragment was cut with XbaI to generate a 9.8 kb fragment. This fragment was then cut with XmaIII to generate a 3.1 kb fragment. The 3.1 kb XmaIII fragment, which contained the gB gene, had XbaI linkers attached to its 5' and 3' termini.

An adenovirus type 5 plasmid, pAd5 Bam-B, which contained the 59.5 - 100 mu region of the Ad5 adenovirus genome cloned into the BamHI site of pBR322 [See R.L. Berkner et al, Nucl. Acids Res., 11:6003-20 (1983) and M.E. Morin et al, cited above] was digested with XbaI to remove the 78.5 mu - 84.7 mu sequences of the Ad5 genome. The 78.5 to 84.7 mu deletion removes most of the coding region of the E3 transcription unit of Ad5 but leaves the E3 promoter intact. The XbaI-linked 3.1 kb fragment of CMV containing the gB gene was inserted into this XbaI site of pAd5 Bam-B. See Fig. 1A for a diagrammatic illustration of the above description.

To generate recombinant virus, the 0-76 mu fragment of wild-type Ad5 virus was isolated by digesting the viral DNA with EcoRI [See U. Petterson et al, J. Mol. Biol., 73:125-30 (1973)]. This fragment was cotransfected with the 59.5 -100 mu BamHI fragment of pAd5 Bam-B containing the gB gene as described above into human embryonic kidney 293 cells, available from ATCC. The Ad5/gB recombinant was generated by overlap recombination between the viral and plasmid sequence as illustrated in Fig. 1B.

The gB recombinant virus was plaque purified on human lung carcinoma A549 cells [ATCC] using standard procedures. vViruses containing both orientations, of the gB gene, as determined by Southern blotting, were isolated.

The recombinant containing the gB gene in the same 5' to 3' direction as the adenovirus E3 promoter of the adenovirus type 5 strain is under the transcriptional control of the E3 promoter. The plaque purified recombinant virus retains the cloning XbaI sites. The above-described cloned gB gene is devoid of its natural promoter according to the DNA sequence of gB identified in Spaete et al, (1987), cited above.

### Example 2 - Production of the gB Subunit

The adenovirus gB plasmid construct and the Ad5 mu 0-76 DNA of Example 1 were cotransfected into 293 cells, human cells transformed by adenovirus 5 early genes [See Graham et al, J. Gen. Virol., 36:59-72 (1977)] employing conventional procedures. This transfection generated a functional recombinant virus by homologous overlap recombination as shown in Fig. 1.

Southern blot analysis confirmed the presence of an Ad5/gB recombinant virus which was subsequently purified by plaque purification using standard procedures.

The recombinant virus expresses gB subunit protein as determined by immunofluorescence on fixed cells and by Western blot using monospecific guinea pig antiserum and monoclonal antibodies to gB protein. [See, e.g., T. Maniatis et al, cited above.]

### Example 3 - Immunization Experiments

Immunization experiments were performed with the vaccine composition of Example 2 using a Syrian hamster model of adenovirus infection, as described in Morin et al, cited above, and Hjorth et al, Arch. Virol., 100:279-83 (1988). Briefly described, hamsters are inoculated intranasally with 10⁷ to 10⁸ pfu of Ad5/gB recombinant virus and evaluated for production of immunity to HCMV. The inoculated animals demonstrate immunity to HCMV by the production of neutralizing antibody to HCMV, as detected by a plaque-reduction neutralization assay according to J.L. Waner et al, in "Manual of Clinical Immunology", eds. N.E. Rose and H. Friedman, American Society for Microbiology, Washington, D.C., pp. 473-477 (1976).

Immunization of human subjects either with the subunits produced in vitro by the recombinant virus or with the live recombinant virus are performed according to the protocol described essentially in Collis et al, cited above. The vaccine of this invention is expected to provide analogous results in humans as in the hamster model, i.e. production of neutralizing antibody to HCMV.

The use of other appropriate non-defective adenovirus strains for construction of analogous expression systems to express the HCMV gB gene may be constructed according to the disclosure of the present invention. Additionally, the other subunits of HCMV major glycoprotein complexes, e.g. gcII or gcIII or immediate-early antigens, may be expressed in a non-defective adenovirus recombinant in the same manner as described above for subunit gB.

## Claims (Claims for the following Contracting State(s): AT, BE, CH, DE, DK, FR, GB, IT, LI, LU, NL, SE)

1. A non-defective recombinant adenovirus genetically engineered to contain a DNA sequence encoding a human cytomegalovirus (CMV) subunit protein under the control of an expression control sequence, said adenovirus being capable of expressing said subunit in a host cell or a human.

2. A recombinant adenovirus according to claim 1, characterised in that the adenovirus is a type 5 strain.

3. A recombinant adenovirus according to claim 1 or 2, characterised in that the CMV subunit protein is selected from gB, gcII, gcIII and immediate-early antigens.

4. A vaccine comprising a genetically engineered non-defective recombinant adenovirus according to any of claims 1 to 3 in a pharmaceutical carrier, said vaccine being capable of eliciting an immune response to CMV upon administration to a human.

5. A vaccine according to claim 4, characterised in that it is in unit dose form for oral adminstration in a pharmaceutically acceptable formulation.

6. The use of the adenovirus according to claim 1, 2 or 3 to prepare a pharmaceutical composition which generates an immune response to CMV in vivo.

## Claims (Claims for the following Contracting State(s): ES, GR)

1. A method for preparing a non-defective recombinant adenovirus for use in a subunit vaccine capable of eliciting an immune response to CMV upon administration to a human,
said method for preparing comprising the steps of:
(a) inserting by genetic engineering techniques a DNA sequence encoding a human cytomegalovirus (CMV) subunit protein into an adenovirus strain under the control of an expression control sequence, said adenovirus being capable of expressing said subunit in a host cell;
(b) transfecting said recombinant adenovirus into a host cell;
(c) culturing said host cell in which the recombinant adenovirus expresses the CMV subunit *in vitro*;
(d) isolating therefrom a recombinant CMV subunit protein; and
(e) admixing said subunit protein with a suitable pharmaceutical carrier.

2. A method for preparing a non-defective recombinant adenovirus according to claim 1, wherein said adenovirus is a type 5 strain.

3. A method for preparing a non-defective recombinant adenovirus according to claim 1 or 2, wherein said CMV subunit protein is selected from gB, gcII, gcIII and immediate-early antigens.

4. A method for preparing a non-defective recombinant adenovirus for use in a vaccine capable of eliciting an immune response to CMV upon administration to a human,
said method comprising the steps of:
(a) inserting by genetic engineering techniques a DNA sequence encoding a human cytomegalovirus (CMV) subunit protein into an adenovirus strain under the control of an expression control sequence, said adenovirus being capable of expressing said subunit in a host cell or human; and
(b) admixing said recombinant adenovirus with a pharmaceutically acceptable carrier.

5. A method for preparing a non-defective recombinant adenovirus according to claim 4, wherein said adenovirus is a type 5 strain.

6. A method for preparing a non-defective recombinant adenovirus according to claim 4 or 5, wherein said CMV subunit protein is selected from gB, gcII, gcIII and immediate-early antigens.

7. A method for preparing a non-defective recombinant adenovirus, said method for preparing comprising:
inserting by genetic engineering techniques a DNA sequence encoding a human cytomegalovirus (CMV) subunit protein into an adenovirus strain under the control of an expression control sequence, said adenovirus being capable of expressing said subunit in a host cell or in a human.

8. A method for preparing a non-defective recombinant adenovirus according to claim 7, wherein said adenovirus is a type 5 strain.

9. A method for preparing a non-defective recombinant adenovirus according to claim 7 or 8, wherein said CMV subunit protein is selected from gB, gcII, gcIII and immediate-early antigens.

## Patentansprüche (Patentansprüche für folgende(n) Vertragsstaat(en): AT, BE, CH, DE, DK, FR, GB, IT, LI, LU, NL, SE)

1. Nicht defektes rekombinantes Adenovirus, gentechnisch so verändert, daß es eine für ein Human-Cytomegalievirus (CMV) - Untereinheitsprotein codierende DNA-Sequenz unter der Kontrolle einer Expressionskontrollsequenz enthält, wobei dieses Adenovirus zur Expression dieser Untereinheit in einer Wirtszelle oder im Menschen fähig ist.

2. Rekombinantes Adenovirus nach Anspruch 1, dadurch gekennzeichnet, daß es sich bei dem Adenovirus um einen Stamm des Typs 5 handelt.

3. Rekombinantes Adenovirus nach Anspruch 1 oder 2, dadurch gekennzeichnet, daß das CMV-Untereinheitsprotein aus den Antigenen gB, gcII, gcIII sowie dem unmittelbaren/frühen Antigen ausgewählt ist.

4. Impfstoff mit einem gentechnisch veränderten nicht defekten rekombinanten Adenovirus nach einem der Ansprüche 1 bis 3 in einem pharmazeutischen Träger, wobei dieser Impfstoff fähig ist, bei Verabreichung an den Menschen eine Immunantwort auf CMV zu provozieren.

5. Impfstoff nach Anspruch 4, dadurch gekennzeichnet, daß er in Einzeldosisform zur oralen Verabreichung als pharmazeutisch unbedenkliche Formulierung vorliegt.

6. Verwendung des Adenovirus nach Anspruch 1, 2 oder 3 zur Herstellung einer Arzneimittelzusammensetzung, die eine Immunantwort auf CMV in vivo hervorruft.

## Patentansprüche (Patentansprüche für folgende(n) Vertragsstaat(en): ES, GR)

1. Verfahren zur Herstellung eines nicht defekten rekombinanten Adenovirus zur Verwendung in einem Untereinheitsimpfstoff, der bei Verabreichung an den Menschen fähig ist, eine Immunantwort auf CMV zu provozieren,
wobei dieses Herstellungsverfahren durch die folgenden Schritte gekennzeichnet ist:
(a) gentechnische Insertion einer für ein Human-Cytomegalievirus (CMV) - Untereinheitsprotein codierenden DNA-Sequenz unter der Kontrolle einer Expressionskontrollsequenz in einen Adenovirusstamm, wobei dieses Adenovirus zur Expression dieser Untereinheit in einer Wirtszelle fähig ist;
(b) Transfektion dieses rekombinanten Adenovirus in eine Wirtszeile;
(c) Züchtung dieser Wirtszeile, in der das rekombinante Adenovirus die CMV-Untereinheit exprimiert, *in vitro*;
(d) Isolierung eines rekombinanten CMV-Untereinheitsproteins hieraus; sowie
(e) Zusatz eines geeigneten pharmazeutischen Trägers zu diesem Untereinheitsprotein.

2. Verfahren zur Herstellung eines nicht defekten rekombinanten Adenovirus nach Anspruch 1, dadurch gekennzeichnet, daß es sich bei diesem Adenovirus um einen Stamm des Typs 5 handelt.

3. Verfahren zur Herstellung eines nicht defekten rekombinanten Adenovirus nach Anspruch 1 oder 2, dadurch gekennzeichnet, daß dieses CMV-Untereinheitsprotein aus den Antigenen gB, gcII, gcIII sowie dem unmittelbaren/frühen Antigen ausgewählt ist.

4. Verfahren zur Herstellung eines nicht defekten rekombinanten Adenovirus zur Verwendung in einem Impfstoff, der fähig ist, bei Verabreichung an den Menschen eine Immunantwort auf CMV zu provozieren,
wobei dieses Verfahren durch die folgenden Schritte gekennzeichnet ist:
(a) gentechnische Insertion einer für ein Human-Cytomegalievirus (CMV)- Untereinheitsprotein codierenden DNA-Sequenz unter der Kontrolle einer Expressionskontrollsequenz in einen Adenovirusstamm, wobei dieses Adenovirus zur Expression dieser Untereinheit in einer Wirtszelle oder im Menschen fähig ist; und
(b) Zusatz eines pharmazeutisch unbedenklichen Trägers zu diesem rekombinanten Adenovirus.

5. Verfahren zur Herstellung eines nicht defekten rekombinanten Adenovirus nach Anspruch 4, dadurch gekennzeichnet, daß es sich bei diesem Adenovirus um einen Stamm des Typs 5 handelt.

6. Verfahren zur Herstellung eines nicht defekten rekombinanten Adenovirus nach Anspruch 4 oder 5, dadurch gekennzeichnet, daß dieses CMV-Untereinheitsprotein aus den Antigenen gB, gcII, gcIII sowie dem unmittelbaren/frühen Antigen ausgewählt ist.

7. Verfahren zur Herstellung eines nicht defekten rekombinanten Adenovirus, wobei dieses Herstellungsverfahren durch folgendes gekennzeichnet ist:
gentechnische Insertion einer für ein Human-Cytomegalievirus (CMV) - Untereinheitsprotein codierenden DNA-Sequenz unter der Kontrolle einer Expressionskontrollsequenz in einen Adenovirusstamm, wobei dieses Adenovirus zur Expression dieser Untereinheit in einer Wirtszelle oder im Menschen fähig ist.

8. Verfahren zur Herstellung eines nicht defekten rekombinanten Adenovirus nach Anspruch 7, dadurch gekennzeichnet, daß es sich bei diesem Adenovirus um einen Stamm des Typs 5 handelt.

9. Verfahren zur Herstellung eines nicht defekten rekombinanten Adenovirus nach Anspruch 7 oder 8, dadurch gekennzeichnet, daß dieses CMV-Untereinheitsprotein aus den Antigenen gB, gcII, gcIII sowie dem unmittelbaren/frühen Antigen ausgewählt ist.

## Revendications (Revendications pour l'(les) Etat(s) contractant(s) suivant(s): AT, BE, CH, DE, DK, FR, GB, IT, LI, LU, NL, SE)

1. Adénovirus recombinant non défectif génétiquement manipulé pour contenir une séquence d'ADN codant pour la protéine de sous-unité du cytomégalovirus (CMV) humain sous le contrôle d'une séquence de contrôle d'expression, ledit adénovirus étant susceptible d'exprimer ladite sous-unité dans une cellule hôte ou chez l'homme.

2. Adénovirus recombinant selon la revendication 1, caractérisé en ce que l'adénovirus est une souche du type 5.

3. Adénovirus recombinant selon la revendication 1 ou 2, caractérisé en ce que La protéine de sous-unité du CMV est choisie parmi gB, gcII, gcIII et des antigènes immédiats-précoces.

4. Vaccin comprenant un adénovirus recombinant non défectif génétiquement manipulé, selon l'une quelconque des revendications 1 à 3, dans un support pharmaceutique, ledit vaccin étant susceptible d'induire une réponse immunitaire vis-à-vis du CMV lors de l'administration à l'homme.

5. Vaccin selon la revendication 4, caractérisé en ce qu'il est sous forme de dose unitaire pour l'administration par voie orale dans une formulation pharmaceutiquement acceptable.

6. Utilisation de l'adénovirus selon la revendication 1, 2 ou 3, pour préparer une composition pharmaceutique engendrant une réponse immunitaire vis-à-vis du CMV in vivo.

## Revendications (Revendications pour l'(les) Etat(s) contractant(s) suivant(s): ES, GR)

1. Procédé de préparation d'un adénovirus recombinant non défectif, destiné à être utilisé dans un vaccin de sous-unité susceptible d'induire une réponse imnunitaire vis-à-vis du CMV lors de l'administration à l'homme,
ledit procédé de préparation comprenant les étapes consistant à:
(a) insérer par des techniques de génie génétique une séquence d'ADN codant pour une protéine de sous-unité du cytomégalovirus (CMV) humain dans une souche d'adénovirus sous le contrôle d'une séquence de contrôle d'expression, ledit adénovirus étant susceptible d'exprimer ladite sous-unité dans une cellule hôte;
(b) transfecter ledit adénovirus recombinant dans une cellule hôte;
(c) cultiver ladite cellule hôte dans laquelle l'adénovirus recombinant exprime la sous-unité de CMV *in vitro*;
(d) en isoler une protéine de sous-unité du CMV recombinante; et
(e) mélanger ladite protéine de sous-unité avec un support pharmaceutique adapté.

2. Procédé de préparation d'un adénovirus recombinant non défectif selon La revendication 1, caractérisé en ce que ledit adénovirus est une souche du type 5.

3. Procédé de préparation d'un adénovirus recombinant non défectif selon la revendication 1 ou 2, caractérisé en ce que ladite protéine de sous-unité du CMV est choisie parmi gB, gcII, gcIII et des antigènes immédiats-précoces.

4. Procédé de préparation d'un adénovirus recombinant non défectif, destiné à être utilisé dans un vaccin susceptible d'induire une réponse immunitaire vis-à-vis du CMV lors de l'administration à l'homme,
ledit procédé de préparation comprenant les étapes consistant à:
(a) insérer par des techniques de génie génétique une séquence d'ADN codant pour une protéine de sous-unité du cytomégalovirus (CMV) humain dans une souche d'adénovirus sous Le contrôle d'une séquence de contrôle d'expression, ledit adénovirus étant susceptible d'exprimer ladite sous-unité dans une cellule hôte ou chez l'homme; et
(b) mélanger ledit adénovirus recombinant avec un support pharmaceutique acceptable.

5. Procédé de préparation d'un adénovirus recombinant non défectif selon la revendication 4, caractérisé en ce que ledit adénovirus est une souche du type 5.

6. Procédé de préparation d'un adénovirus recombinant non défectif selon la revendication 4 ou 5, caractérisé en ce que ladite protéine de sous-unité du CMV est choisie parmi gB, gcII, gcIII et des antigènes immédiats-précoces.

7. Procédé de préparation d'un adénovirus recombinant non défectif, ledit procédé de préparation comprenant:
l'insertion par des techniques de génie génétique d'une séquence d'ADN codant pour une protéine de sous-unité du cytomégalovirus (CMV) humain dans une souche d'adénovirus sous le contrôle d'une séquence de contrôle d'expression, ledit adénovirus étant susceptible d'exprimer ladite sous-unité dans une cellule hôte ou chez l'homme.

8. Procédé de préparation d'un adénovirus recombinant non défectif selon la revendication 7, caractérisé en ce que ledit adénovirus est une souche du type 5.

9. Procédé de préparation d'un adénovirus recombinant non défectif selon la revendication 7 ou 8, caractérisé en ce que ladite protéine de sous-unité du CMV est choisie parmi gB, gcII, gcIII et des antigènes immédiats-précoces.
